# EUROPÄISCHE PATENTSCHRIFT

(11) **EP 0 894 495 B1**
(45) Veröffentlichungstag und Bekanntmachung des Hinweises auf die Patenterteilung: **17.01.2001**
(21) Anmeldenummer: 98250231.2
(22) Anmeldetag: 24.06.1998
(51) Int. Cl.: A61K 9/00, A61K 9/20

(54) **Bioadhäsive Tablette mit Vertiefungen**
Bioadhesive embossed tablets
Comprimes bioadhesifs avec des creux

(30) Priorität: 30.07.1997 DE 19734538
(43) Veröffentlichungstag der Anmeldung: 03.02.1999
(73) Patentinhaber: Jenapharm GmbH & Co. KG, 07745 Jena (DE)
(72) Erfinder: Timpe, Carsten, Dr., 37299 Weissenborn (DE); Dittgen, Michael, Prof. Dr., 99510 Apolda (DE); Grawe, Detlef, 99510 Kleinromstedt (DE); Schumacher, Jochen, 07745 Jena (DE); Zimmermann, Holger, Dr., 98693 Il menau-Roda (DE); Hoffmann, Herbert, Prof. Dr., 07749 Jena (DE)
(74) Vertreter: Leybach, Holger Hugo

(56) Entgegenhaltungen:
- WO-A-87/04342
- DE-U- 9 200 765

## Beschreibung

Die Erfindung betrifft eine bioadhäsive Tablette, enthaltend mindestens einen bioadhäsiven Hilfsstoff und mindestens ein Gleitmittel, wobei mindestens eine Oberfläche der Tablette konzentrische oder parallel verlaufende, geradlinige und/oder geschwungene Vertiefungen aufweist, ein Verfahren zur Herstellung dieser bioadhäsiven Tabletten sowie Arzneimittel in Form solcher bioadhäsiven Tabletten.

Seit etwa 10 Jahren sind bioadhäsive Arzneimittel bekannt, die zeitweise an biologischem Gewebe haften und dabei die Wirkstoffe freisetzen. Von diesen bio- oder mucosaadhäsiven Arzneimitteln erwartet man aufgrund der Schleimhauthaftung eine Lösung von Bioverfügbarkeitsproblemen, die aus einer zu kurzen Verweilzeit der Wirkstoffe am Resorptionsort erwachsen. Insbesondere können bioadhäsive Arzneimittel an bestimmten Schleimhäuten, z.B. des Magen-Darm-Traktes, den Wirkstoff bevorzugt zur Resorption bringen.

Die Haftung dieser Arzneiformen am Resorptionsort wird durch bioadhäsive Polymere vermittelt, von denen Hydrogelbildner, wie Cellulosederivate (Hydroxyethylcellulose (HEC), Natriumcarboxymethylcellulose (NaCMC)), quervernetzte Polyacrylsäure (Carbopol®) und andere Carboxyvinylpolymere (Eudragit®), Tragant und Alginate die stärkste Wirkung haben sollen. In einer Übersicht (Duchene, D.; Touchard,F; Peppas,N.A.: Drug Dev. Ind. Pharm. 14, 283 (1988) ) wurden bioadhäsive Arzneimittel, z.B. Tabletten, Filme, Pflaster, Gele und Kapseln beschrieben, die lokale oder systemische Wirkungen entfalten und die hauptsächlich oral (buccal) aber auch peroral (Magen-Darm-Trakt), periocular, nasal, vaginal oder rektal appliziert werden können. Als bioadhäsive Polymere für diese Arzneimittel wurden Carboxyvinylpolymere (Carbopol®, Polycarbophil®) und Cellulosederivate (Hydroxypropylcellulose (HPC), HEC, NaCMC) bzw. Mischungen beider Komponenten genannt. Insbesondere eignen sich Carboxyvinylpolymere und von diesen abgeleitete Verbindungen als bioadhäsive Polymere (Peppas,N.A.; Buri,P.A.; J. Controlled Release 2, 257 (1985); Park, K.; Cooper, S.L.; Robinson, J.R.: Hydrogels Med. Pharm. 3, 151 (1987)).

DD 282 551 A5 betrifft ein Verfahren zur Herstellung von bioadhäsiven Arzneimitteln, beispielsweise von Tabletten, Granulaten, Schleimen, die ein in Wasser quellbares Copolymerisat mit höchstens 25 Massenanteilen in % an Monomeren mit Carboxylfunktionen enthalten, das sich im wesentlichen durch einen Fremdstoffgehalt von maximal 0,56 Massenanteilen in % sowie weiterhin dadurch auszeichnet, daß es unter Verwendung von maximal 0,5 Massenanteilen in % eines anionischen Emulgators und von maximal 0,06 Massenanteilen in % eines Initiators hergestellt wird.

Die bisher bekannten festen bioadhäsiven Arzneimittel zerfallen in der Regel nur langsam. Oft ist vorgesehen, daß die unveränderte Arzneiform, beispielsweise Mikropartikeln, vor allem aber Zweischicht- und Mehrschichttabletten, mit dem Gewebe in Kontakt gebracht wird. Dabei steht für den Wirkstoffdurchtritt, die Resorption, nur eine relativ eng begrenzte Fläche zur Verfügung. Diese Fläche kann sich mit beginnender Quellung der jeweiligen Arzneiform und abhängig vom eingesetzten bioadhäsiven Polymer noch weiter verringern. Dies ist beispielsweise der Fall bei einer aus wirkstoffhaltigem Kern und bioadhäsivem Mantel bestehenden quellbaren bioadhäsiven Manteltablette (Ishida, M.; Machida, Y.; Nambu, N.; Nagai, T.: Chem. Pharm. Bull. 29, 810 (1981)).

Auch kürzlich vorgestellte bioadhäsive Mikrospheren enthalten den Arzneistoff im Kern und sind mit einer Schicht eines bioadhäsiven Polymers überzogen (Junginger, H.E.; Lehr, C.M.: Dtsch. Apoth. Ztg. 130, 791 (1990)). Bei der Quellung der Mikrospheren kann sich eine Diffusionsschicht ausbilden, die den Arzneistoffdurchtritt erschwert.

Somit werden bioadhäsive Tabletten, beispielsweise Buccaltabletten, meist als Zwei- und Mehrschichttabletten mit kontrollierter Wirkstofffreigabe beschrieben (Junginger, H.E.; de Vries, M.E.; Bodde, H.E.: Dtsch. Apoth. Ztg. 131, 1337 (1991)). Diese Tabletten haften zunächst aufgrund kapillarer Effekte, später, nach Hydratation und Quellung, durch Bindungen, die durch das interdiffundierende bioadhäsive Polymer bedingt sind.

Andere bioadhäsive Tabletten, speziell Buccaltabletten (Veillard, M. in: Gurny, R.; Junginger, H.E. (Eds.) "Bioadhesion - Possibilities and Future Trends", S. 124, APV-Paperback, Wiss. Verlagsgesellschaft, Stuttgart 1990), quellen nur eingeschränkt und sollen dadurch besonders zu einer Langzeithaftung am Gewebe befähigt sein.

Ausnahmsweise werden auch bioadhäsive Schleime, also wäßrige Zubereitungen (Robinson, J.R.: S.T.P. Pharma 5, 839 (1989)), oder eine zum Schleim aufquellende Tablette (DE 4 139 883 A1) angewandt. Diese haften im allgemeinen schwächer am Gewebe als feste Arzneiformen oder deren Zerfallsprodukte (Saettone, M.F.; Chetoni, P.; Torracca, M.T.; Burgalassi, S.; Giannaccini, B.: Int. J. Pharm. 51, 203 (1989)) und sie haben die bekannten Nachteile der Anfälligkeit gegenüber Mikroorganismen und der Instabilität enthaltener Wirkstoffe.

Darüber hinaus firdet man eine Reihe rasch zerfallender Arzneimittel, die aufgrund dieser Tatsache den erfindungsgemäßen Tabletten nicht nahe kommen. So bezieht sich U.S.-PS 50 07 790 auf Tabletten und Kapseln mit quellbaren Polymeren zur Anwendung im Magen-Darm-Trakt.

In der U.S.-PS 48 86 669 sind Tabletten beschrieben, die wirkstoffhaltige Mikropartikel, einen Zerfallhilfsstoff und einen Quellstoff enthalten. Nach dem raschen Zerfall der Tabletten in Wasser resultiert eine homogene Suspension hoher Viskosität. Bekannt ist jedoch, daß zwischen Viskosität und Bioadhäsion kein direkter Zusammenhang besteht (Dittgen,M.; Oestereich,S.; Dittrich,F.: Pharmazie 44, 460 (1989), Satoh,K.; Takayama,K.; Machida,Y.; Suzuki,Y.; Nakagaki,M.; Nagai,T.: Chem.Pharm.Bull. 37, 1366 (1989)).

Aufgabe der vorliegenden Erfindung ist es, die Nachteile der bisher bekannten bioadhäsiven Tabletten zu überwinden. Insbesondere ist es eine Aufgabe der Erfindung, den Durchtritt der enthaltenen Wirkstoffe durch die Schleimhaut zu verbessern.

Die Aufgabe wird erfindungsgemäß durch eine bioadhäsive Tablette nach Anspruch 1, enthaltend mindestens einen bioadhäsiven Hilfsstoff und mindestens ein Gleitmittel, wobei mindestens eine Oberfläche der Tablette konzentrische oder parallel verlaufende, geradlinige und/oder geschwungene Vertiefungen aufweist, gelöst.

Die erfindungsgemäßen bioadhäsiven Tabletten zerfallen, so daß sie oder ihre Zerfallsprodukte den Durchtritt der in ihnen enthaltenen Wirkstoffe durch die Schleimhaut durch Quellung nicht behindern. Die erfindungsgemäße Tablette stellt eine feste, jedoch im Organismus bioadhäsive Arzneiform dar, in der die Wirkstoffe stabil und vor einem mikrobiellen Angriff geschützt bereitgestellt werden und aus der heraus sie über eine ausgedehnte Gewebefläche des Zielorgans resorbiert werden können.

Die erfindungsgemäßen bioadhäsiven Tabletten setzen die enthaltenen Wirkstoffe fast vollständig frei, fördern deren Übertritt in das Gewebe und gehen dabei keine unerwünschten Wechselwirkungen mit dem biologischen Gewebe ein.

Vorteilhaft ist es, daß der bioadhäsive Hilfsstoff eine Substanz ist, die bei Kontakt mit der Schleimhaut einen Haftkontakt entwickelt, wie eine Cellulose, ein Cellulosederivat, ein Carboxyvinylpolymer, ein Derivat eines Carboxyvinylpolymeren, ein Lectin oder ein Naturstoff oder Mischungen aus diesen Substanzen.

Es ist weiterhin vorteilhaft, daß das Gleitmittel die Tablettierung kohäsiver Mischungen ermöglicht, wie Talk, eine Metallseife, eine Fettsäure oder Mischungen aus diesen Substanzen.

Außerdem ist es erfindungsgemäß vorteilhaft, daß der Anteil der Tablette an bioadhäsivem Hilfsstoff zwischen 5 und 65 Gew%, vorzugsweise zwischen 10 und 45 Gew% beträgt, und der Anteil an Gleitmittel zwischen 0,05 und 5 Gew%, vorzugsweise zwischen 1 und 3 Gew% beträgt, und daß das Verhältnis von Gleitmittel zu bioadhäsivem Hilfsstoff zwischen 1:1300 und 1:1, vorzugsweise zwischen 1:10 und 1:15, beträgt.

Besonders vorteilhaft ist es, daß die Oberfläche der erfindungsgemäßen bioadhäsiven Tablette 2 bis 12, vorzugsweise 3 bis 5, konzentrisch oder parallel verlaufende geradlinige und/oder geschwungene Vertiefungen aufweist, die eine Tiefe von 0,1 mm bis 1,0 mm, vorzugsweise von 0,3 mm bis 0,6 mm, gerechnet von der Tablettenoberfläche, und untereinander einen Abstand von 0,5 bis 5 mm, vorzugsweise 1,5 bis 2,5 mm haben. Die Vertiefungen können kerbenförmig ausgestaltet sein und die Flanken der Kerben können kreisförmig gewölbt sein, wobei der Radius von 0,1 bis 0,9 mm, vorzugsweise 0,5 mm, und/oder der Öffnungswinkel der Kerbe 45 bis 135 °, vorzugsweise 90 ° beträgt.

Ein Gegenstand der vorliegenden Erfindung ist ferner ein Verfahren zur Herstellung bioadhäsiver Tabletten, umfassend das Mischen mindestens eines bioadhäsiven Hilfsstoffs und mindestens eines Gleitmittel und Pressen einer Tablette, wobei mindestens eine Oberfläche der Tablette konzentrische oder parallel verlaufende, geradlinige und/oder geschwungene Vertiefungen aufweist.

Bevorzugt ist ein erfindungsgemäßes Verfahren zur Herstellung bioadhäsiver Tabletten, welches dadurch gekennzeichnet ist, daß der bioadhäsive Hilfsstoff eine Substanz ist, die bei Kontakt mit der Schleimhaut einen Haftkontakt entwickelt, wie eine Cellulose, ein Cellulosederivat, ein Carboxyvinylpolymer, ein Derivat eines Carboxyvinylpolymeren, ein Lectin oder ein Naturstoff oder Mischungen aus diesen Substanzen.

Bevorzugt ist auch Verfahren zur Herstellung bioadhäsiver Tabletten, welches dadurch gekennzeichnet ist, daß das Gleitmittel die Tablettierung kohäsiver Mischungen ermöglicht, wie Talk, eine Metallseife, eine Fettsäure oder Mischungen aus diesen Substanzen.

Besonders bevorzugt ist ein erfindungsgemäßes Verfahren zur Herstellung bioadhäsiver Tabletten, wobei der Anteil der Tablette an bioadhäsivem Hilfsstoff zwischen 5 und 65 Gew%, vorzugsweise zwischen 10 und 45 Gew% beträgt, und der Anteil an Gleitmittel zwischen 0,05 und 5 Gew%, vorzugsweise zwischen 1 und 3 Gew% beträgt, und daß das Verhältnis von Gleitmittel zu bioadhäsivem Hilfsstoff zwischen 1:1300 und 1:1, vorzugsweise zwischen 1:10 und 1:15, beträgt.

Insbesondere bevorzugt ist ein Verfahren zur Herstellung bioadhäsiver Tabletten, bei dem die Oberfläche 2 bis 12, vorzugsweise 3 bis 5, konzentrisch oder parallel verlaufende geradlinige und/oder geschwungene Vertiefungen aufweist, die eine Tiefe von 0,1 mm bis 1,0 mm, vorzugsweise von 0,3 mm bis 0,6 mm, gerechnet von der Tablettenoberfläche, und untereinander einen Abstand von 0,5 bis 5 mm, vorzugsweise 1,5 bis 2,5 mm haben und wobei die Vertiefungen kerbenförmig ausgestaltet sind und wobei die Flanken der Kerben kreisförmig gewölbt sind, mit einem Radius von 0,1 bis 0,9 mm, vorzugsweise 0,5 mm, und/oder der Öffnungswinkel der Kerbe 45 bis 135 °, vorzugsweise 90 ° beträgt.

Ein weiterer Gegenstand der vorliegenden Erfindung sind Arzneimittel, welches in Form der erfindungsgemäßen bioadhäsiven Tablette vorliegen und Wirkstoffe der unterschiedlichsten Indikationsgebiete enthalten.

Gegenstand der vorliegenden Erfindung sind insbesondere Arzneimittel in Form einer erfindungsgemäßen bioadhäsiven Tablette, enthaltend als Wirkstoff Antirheumatika, Analgetika, Antiparkinsonmittel, β-Rezeptorenblocker, Sexualhormone, Kontrazeptiva, Herz-Kreislaufmittel, Schlaf- und Hypophysenhormone, Antidiabetika, Immuntherapeutika oder Antikoagulanzien.

Die erfindungsgemäßen bioadhäsiven Tabletten lassen sich in an sich bekannter Weise herstellen. Es können beliebige Wirkstoffe, insbesondere Arzneistoffe, durch den Zusatz eines bioadhäsiven Hilfsstoffes, eines Gleitmittels und gegebenenfalls weiterer für die Tablettierung üblicher Hilfsstoffe in einfacher Technologie zu Tabletten gepreßt werden, die an der Schleimhaut haften. Die bioadhäsive Tablette soll im Organismus sofort nach Kontakt mit der Schleimhaut an dieser haften, eine möglichst große Kontaktfläche zur Schleimhaut ausbilden, und ausschließlich Hilfsstoffe enthalten, die toxikologisch unbedenklich sind und die Resorption der Wirkstoffe begünstigen.

Als Arzneistoffe kommen alle diejenigen in Frage, für die eine gesicherte oder gesteuerte Bioverfügbarkeit sinnvoll ist. Dabei werden bezüglich der Wirkungsqualität keine Festlegungen getroffen, so daß beispielsweise Antirheumatika, Analgetika, Parkinsonmittel, β-Rezeptorenblocker, Sexualhormone und insbesondere Kontrazeptiva, Herz-Kreislaufmittel, Schlaf- und Hypophysenhormone, Antidiabetika, Immuntherapeutika, Antikoagulanzien und weitere Arzneistoffe nach dem erfindungsgemäßen Verfahren zu bioadhäsiven Tabletten verarbeitet werden können. Auch bezüglich des angestrebten Resorptionsortes der Arzneiform werden ausdrücklich keine Festlegungen getroffen. Vorzugsweise dient die Erfindung der Herstellung bioadhäsiver Peroralia und Vaginalia.

Gegenstand der vorliegenden Erfindung sind somit Arzneimittel zur oralen, peroralen, rektalen oder vaginalen, die neben üblichen Träger- und Verdünnungsmitteln eine pharmazeutisch wirksame Verbindung als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen, rektalen oder vaginalen Applikation geeignet ist. Entsprechende geeignete Verfahren sind beispielsweise beschrieben in "Hagers Handbuch der pharmazeutischen Praxis, 4. Auflage, 1967-89, Springer Verlag, Berlin".

Entsprechende Tabletten können beispielsweise durch Mischen des Wirkstoffs mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Die Erfindung wird nachfolgend in Ausführungsbeispielen anhand der zugehörigen Zeichnungen näher erläutert. Es zeigen:
- Figur 1: eine bioadhäsive Tablette im Querschnitt, in der Aufsicht mit konzentrischen Vertiefungen und in der Aufsicht mit parallel verlaufenden, geradlinigen und/oder geschwungenen Vertiefungen,
- Figur 2: eine bioadhäsive Tablette am Resorptionsort und
- Figur 3: die Darstellung der Bioadhäsion verschiedener bioadhäsiver Tabletten mit unterschiedlicher Masse an Gleitmittel (C₁, mg) und bioadhäsivem Hilfsstoff (C₂, mg).

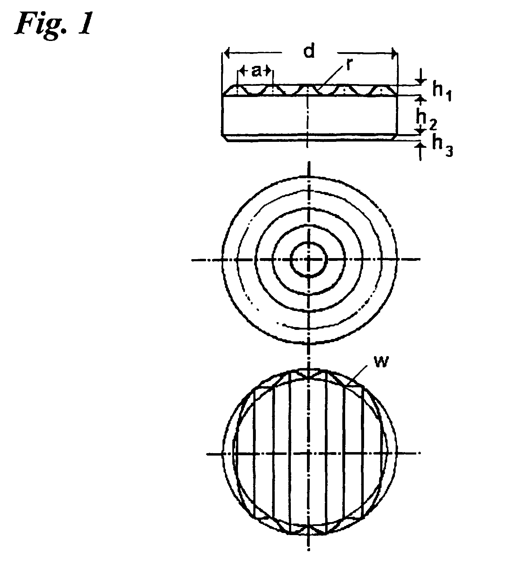

Figur 1 stellt die erfindungsgemäße Lösung der Aufgabe an Hand eines Ausführungsbeispiels für eine erfindungsgemäße bioadhäsive Tablette dar. Die Tablette zeichnet sich dadurch aus, daß eine rauhe Oberfläche gebildet wird, indem beim Pressen der Tablette mindestens auf einer Seite derselben Vertiefungen erzeugt werden. Überraschenderweise wurde gefunden, daß insbesondere runde und/oder winklige, insbesondere rechtwinklige Vertiefungen, deren Anzahl und Abstand zueinander in einem ausgewogenen Verhältnis zur Oberfläche steht, eine geeignete rauhe Oberfläche darstellen. Insbesondere haben sich folgende Abmessungen der Vertiefungen und übrigen Parameter der Tablette als zweckmäßig erwiesen:
- a: Abstand zwischen zwei Vertiefungen, 0,5 bis 5 mm, vorzugsweise 1,5 bis 2,5 mm,
- d: Durchmesser der Tablette, 3 bis 15 mm, vorzugsweise 7 bis 9 mm,
- h₁: Tiefe der Vertiefungen, 0,1 mm bis 1,0 mm, vorzugsweise 0,3 mm bis 0,6 mm,
- h₂: Steghöhe der Tablette, 0,1 bis 8 mm, vorzugsweise 0,2 bis 0,4 mm
- h₃: Höhe der Facette, 0,05 mm bis 2,0 mm, vorzugsweise 0,1 mm bis 1,0 mm,
- r: Radius der Vertiefung, 0,1 bis 0,9 mm, vorzugsweise 0,5 mm
- w: Winkel der Vertiefung, 45 bis 135°, vorzugsweise 90°.

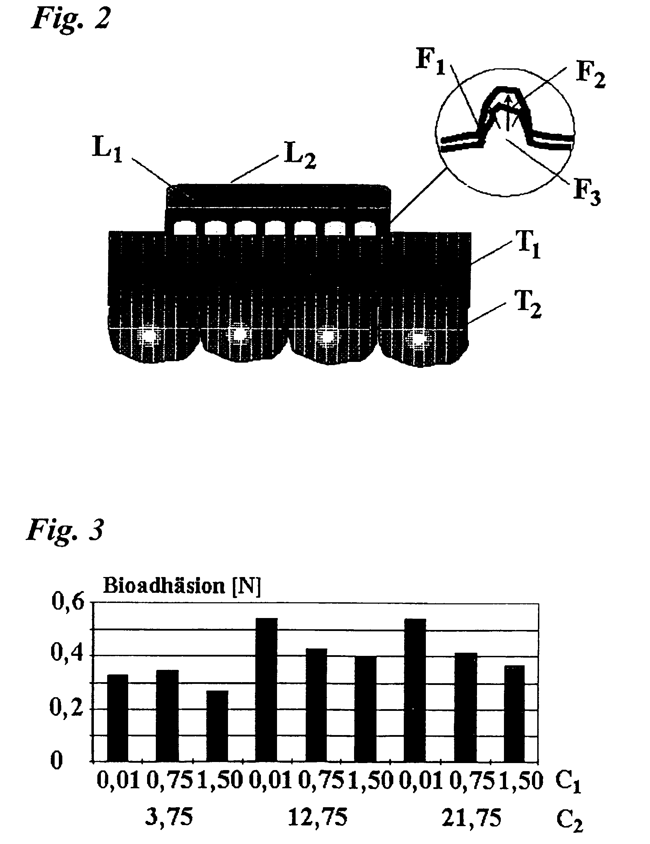

Figur 2 stellt die Wirkweise der erfindungsgemäßen Tablette am Wirkort bzw. Ort der Resorption dar. Durch die erfindungsgemäß gestaltete Oberfläche der Tablette haftet diese unmittelbar nach Kontakt mit der Schleimhaut an dieser. Hierbei wirken bedingt durch die abgestimmte Geometrie der Vertiefungen Haft- (F₁), Quellungs- (F₂), und Grenzflächenkräfte (F₃). Der Arzneistoff kann, sofern er nicht ganz oder teilweise in der bioadhäsiven Schicht (L₁) enthalten ist, dieselbe in den schleimhautfernen Bereichen der Vertiefungen leicht durchdringen, wodurch eine hohe Bioverfügbarkeit gewährleistet wird.

Figur 3 stellt die Abhängigkeit der Bioadhäsion, gemessen auf einer Dialysemembran (Visking 36/32 der Roth GmbH & Co. D-Karlsruhe, Methode: Herold, K., Entwicklung, Untersuchung und Optimierung einer bukkalen, bioadhäsiven Progesteron-Zweischichttablette, Diplomarbeit, Univ. Halle (Pharmazie) 1997), vom Verhältnis des Gleitmittels zum bioadhäsiven Hilfsstoff dar. Eine erfindungsgemäße Tablette enthält notwendigerweise einen bioadhäsiven Hilfsstoff und ein Gleitmittel, welches die Tablettierung ermöglicht. Diese beiden Hilfsstoffe stellen Antagonisten dar. Der bioadhäsive Hilfsstoff klebt und haftet leicht. Das Gleitmittel verhindert das Kleben und Haften. Außerdem werden die gewünschten Hafteffekte des in der jeweils erforderlichen Menge zugesetzten bioadhäsiven Hilfsstoffe durch das zugesetzte Gleitmittel abgeschwächt. Überraschenderweise wurde gefunden, daß in einem bestimmten und relativ begrenzten Verhältnis von Gleitmittel und bioadhäsivem Hilfsstoff im Bereich von 1:1300 bis 1:1, vorzugsweise jedoch in einem Bereich von 1:10 und 1:15, sowohl die Herstellung der Tabletten gelingt als auch der gewünschte bioadhäsive Effekt an der Schleimhaut eintritt.

Die folgenden Beispiele erläutern die Erfindung in nicht beschränkender Weise.

### Beispiel 1:

Bioadhäsive Tablette mit Progesteron (orale Anwendung)

| | |
|---|---|
| Progesteron | 20,0 g |
| Cyclodextrin | 100,0 g |
| Mannitol | 100,0 g |
| Bindemittel | 5,5 g |

Die Bestandteile werden gemischt und in bekannter Weise zu Tabletten verpreßt. Die Bioadhäsion der Tabletten, ex vivo tensiometrisch nach einer Kontaktzeit von 5 Minuten auf frischem Schweinedünndarm bestimmt (Dittrich, F., Entwicklung einer neuen bioadhäsiven Tablette zur enteralen und vaginalen Anwendung und Untersuchung einiger ihrer wesentlichen Eigenschaften in vitro und in vivo, Disertation Univ. Greifswald 1982, Seite 64-65), beträgt 0,62 N.

### Beispiel 2:

Bioadhäsive Tablette mit Estradiol (vaginale Anwendung)

| | |
|---|---|
| Estradiol, co-mikronisiert | 50 mg |
| Fructose | 48 mg |
| Lactose-Monohydrat | 15 mg |
| Natriumcarboxymethylcellulose | 15 mg |
| Glycerolpalmitostearinsäureester | 2 mg |

Die Bestandteile werden gemischt und in bekannter Weise zu Tabletten verpreßt. Die Bioadhäsion der Tabletten, ex vivo tensiometrisch nach einer Kontaktzeit von 5 Minuten auf frischem Schweinedünndarm bestimmt (siehe Beispiel 1), beträgt 0,58 N.

### Beispiel 3:

Bioadhäsive Zweischichttablette mit Testosteron (orale Anwendung)

| Wirkstoffschicht: | |
|---|---|
| Testosteron Comikronisat | 50 mg |
| Mannitol | 48 mg |
| Cellactose | 30 mg |
| Hydroxyethylcellulose | 1 mg |
| Talkum | 4 mg |
| Magnesiumstearat | 1 mg |

| Bioadhäsive Schicht: | |
|---|---|
| Mannitol | 18 mg |
| Cellactose | 35 mg |
| Natriumcarboxymethylcellulose | 10 mg |
| Talkum | 1 mg |
| Magnesiumstearat | 1 mg |
| Eisenoxid LMF Rot 30 | 0,05 mg |

Die Bestandteile werden gemischt und in bekannter Weise zu Zweischichttabletten verpreßt. Die Bioadhäsion der Tabletten, ex vivo tensiometrisch nach einer Kontaktzeit der bioadhäsiven Schicht von 5 Minuten auf frischem Schweinedünndarm bestimmt(siehe Beispiel 1), beträgt 0,88 N.

### Beispiel 4:

Bioadhäsive Zweischichttablette mit Dehydroepiandrosteron (rektale Anwendung)

| Wirkstoffschicht: | |
|---|---|
| Dehydroepiandrosteron Comikronisat | 50 mg |
| Lävulose | 52 mg |
| Cellactose | 30 mg |
| Hydroxyethylcellulose | 5 mg |
| Talkum | 4 mg |
| Magnesiumstearat | 1 mg |

| Bioadhäsive Schicht: | |
|---|---|
| Lävulose | 8 mg |
| Cellactose | 45 mg |
| Natriumcarboxymethylcellulose | 15 mg |
| Talkum | 1 mg |
| Magnesiumstearat | 1 mg |
| Eisenoxid LMF Rot 30 | 0,05 mg |

Die Bestandteile werden gemischt und in bekannter Weise zu Zweischichttabletten verpreßt. Die Bioadhäsion der Tabletten, ex vivo tensiometrisch nach einer Kontaktzeit der bioadhäsiven Schicht von 5 Minuten auf frischem Schweinedünndarm bestimmt(siehe Beispiel 1), beträgt 0,36 N.

### Bezugszeichenliste

- a: Abstand zwischen zwei Vertiefungen
- d: Durchmesser der Tablette
- h₁: Tiefe der Vertiefungen
- h₂: Steghöhe der Tablette
- h₃: Höhe der Facette
- r: Radius der Vertiefung
- w: Winkel der Vertiefung

- F₁: Adhäsion
- F₂: Quellung
- F₃: Grenzflächenspannung
- L₁: bioadhäsive Schicht
- L₂: arzneistoffhaltige Schicht
- T₁: Schleimschicht
- T₂: Epithel

## Patentansprüche

1. Bioadhäsive Tablette, enthaltend mindestens einen bioadhäsiven Hilfsstoff und mindestens ein Gleitmittel, wobei das Verhältnis von Gleitmittel zu bioadhäsivem Hilfsstoff von 1:1300 bis 1:1 beträgt und mindestens eine Oberfläche der Tablette konzentrische oder parallel verlaufende, geradlinige und/oder geschwungene Vertiefungen aufweist.

2. Bioadhäsive Tablette gemäß Anspruch 1, dadurch gekennzeichnet, daß der bioadhäsive Hilfsstoff eine Substanz ist, die bei Kontakt mit der Schleimhaut einen Haftkontakt entwickelt, wie eine Cellulose, ein Cellulosederivat, ein Carboxyvinylpolymer, ein Derivat eines Carboxyvinylpolymeren, ein Lectin oder ein Naturstoff oder Mischungen aus diesen Substanzen.

3. Bioadhäsive Tablette gemäß Anspruch 1 oder 2, dadurch gekennzeichnet, daß das Gleitmittel die Tablettierung kohäsiver Mischungen ermöglicht, wie Talk, eine Metallseife, eine Fettsäure oder Mischungen aus diesen Substanzen.

4. Bioadhäsive Tablette gemäß mindestens einem der voranstehenden Ansprüche, dadurch gekennzeichnet, daß der Anteil der Tablette an bioadhäsivem Hilfsstoff zwischen 5 und 65 Gew%, vorzugsweise zwischen 10 und 45 Gew% beträgt, und der Anteil an Gleitmittel zwischen 0,05 und 5 Gew%, vorzugsweise zwischen 1 und 3 Gew% beträgt, und daß das Verhältnis von Gleitmittel zu bioadhäsivem Hilfsstoff vorzugsweise von 1:10 bis 1:15 beträgt.

5. Bioadhäsive Tablette gemäß mindestens einem der voranstehenden Ansprüche, dadurch gekennzeichnet, daß die Oberfläche 2 bis 12, vorzugsweise 3 bis 5, konzentrisch oder parallel verlaufende geradlinige und/oder geschwungene Vertiefungen aufweist, die eine Tiefe von 0,1 mm bis 1,0 mm, vorzugsweise von 0,3 mm bis 0,6 mm, gerechnet von der Tablettenoberfläche, und untereinander einen Abstand von 0,5 bis 5 mm, vorzugsweise 1,5 bis 2,5 mm haben und wobei die Vertiefungen kerbenförmig ausgestaltet sind und wobei die Flanken der Kerben kreisförmig gewölbt sind, mit einem Radius von 0,1 bis 0,9 mm, vorzugsweise 0,5 mm, und/oder der Öffnungswinkel der Kerbe 45 bis 135 °, vorzugsweise 90 ° beträgt.

6. Verfahren zur Herstellung bioadhäsiver Tabletten, umfassend das Mischen mindestens eines bioadhäsiven Hilfsstoffs und mindestens eines Gleitmittels und Pressen einer Tablette, wobei das Verhältnis von Gleitmittel zu bioadhäsivem Hilfsstoff von 1:1300 bis 1:1 beträgt und mindestens eine Oberfläche der Tablette konzentrische oder parallel verlaufende, geradlinige und/oder geschwungene Vertiefungen aufweist.

7. Verfahren zur Herstellung bioadhäsiver Tabletten gemäß Anspruch 6, dadurch gekennzeichnet, daß der bioadhäsive Hilfsstoff eine Substanz ist, die bei Kontakt mit der Schleimhaut einen Haftkontakt entwickelt, wie eine Cellulose, ein Cellulosederivat, ein Carboxyvinylpolymer, ein Derivat eines Carboxyvinylpolymeren, ein Lectin oder ein Naturstoff oder Mischungen aus diesen Substanzen.

8. Verfahren zur Herstellung bioadhäsiver Tabletten gemäß Anspruch 6 oder 7, dadurch gekennzeichnet, daß das Gleitmittel die Tablettierung kohäsiver Mischungen ermöglicht, wie Talk, eine Metallseife, eine Fettsäure oder Mischungen aus diesen Substanzen.

9. Verfahren zur Herstellung bioadhäsiver Tabletten gemäß mindestens einem der Ansprüche 6 bis 8, dadurch gekennzeichnet, daß der Anteil der Tablette an bioadhäsivem Hilfsstoff zwischen 5 und 65 Gew%, vorzugsweise zwischen 10 und 45 Gew% beträgt, und der Anteil an Gleitmittel zwischen 0,05 und 5 Gew%, vorzugsweise zwischen 1 und 3 Gew% beträgt, und daß das Verhältnis von Gleitmittel zu bioadhäsivem Hilfsstoff vorzugsweise von 1:10 bis 1:15 beträgt.

10. Verfahren zur Herstellung bioadhäsiver Tabletten gemäß mindestens einem der Ansprüche 6 bis 9, dadurch gekennzeichnet, daß die Oberfläche 2 bis 12, vorzugsweise 3 bis 5, konzentrisch oder parallel verlaufende geradlinige und/oder geschwungene Vertiefungen aufweist, die eine Tiefe von 0,1 mm bis 1,0 mm, vorzugsweise von 0,3 mm bis 0,6 mm, gerechnet von der Tablettenoberfläche, und untereinander einen Abstand von 0,5 bis 5 mm, vorzugsweise 1,5 bis 2,5 mm haben und wobei die Vertiefungen kerbenförmig ausgestaltet sind und wobei die Flanken der Kerben kreisförmig gewölbt sind, mit einem Radius von 0,1 bis 0,9 mm, vorzugsweise 0,5 mm, und/oder der Öffnungswinkel der Kerbe 45 bis 135 °, vorzugsweise 90 ° beträgt.

11. Arzneimittel in Form einer bioadhäsiven Tablette gemäß Anspruch 1, enthaltend als Wirkstoff Antirheumatika, Analgetika, Antiparkinsonmittel, β-Rezeptorenblocker, Sexualhormone, Kontrazeptiva, Herz-Kreislaufmittel, Schlaf- und Hypophysenhormone, Antidiabetika, Immuntherapeutika oder Antikoagulanzien.

## Claims

1. Bioadhesive tablet containing at least one bioadhesive excipient and at least one lubricant, where the ratio of lubricant to bioadhesive excipient is from 1:1300 to 1:1, and at least one surface of the tablet has concentric or parallel, straight-line and/or curved impressions.

2. Bioadhesive tablet according to Claim 1, characterized in that the bioadhesive excipient is a substance which, on contact with the mucosa, develops an adhesive contact, such as a cellulose, a cellulose derivative, a carboxyvinyl polymer, a derivative of a carboxyvinyl polymer, a lectin or a natural substance or mixtures of these substances.

3. Bioadhesive tablet according to Claim 1 or 2, characterized in that the lubricant makes the tableting of cohesive mixtures possible, such as talc, a metallic soap, a fatty acid or mixtures of these substances.

4. Bioadhesive tablet according to at least one of the preceding claims, characterized in that the content of bioadhesive excipient in the tablet is between 5 and 65% by weight, preferably between 10 and 45% by weight, and the content of lubricant is between 0.05 and 5% by weight, preferably between 1 and 3% by weight, and in that the ratio of lubricant to bioadhesive excipient is preferably from 1:10 to 1:15.

5. Bioadhesive tablet according to at least one of the preceding claims, characterized in that the surface has 2 to 12, preferably 3 to 5, concentric or parallel straight-line and/or curved impressions which have a depth of from 0.1 mm to 1.0 mm, preferably from 0.3 mm to 0.6 mm, calculated from the tablet surface, and have a distance apart of from 0.5 to 5 mm, preferably 1.5 to 2.5 mm, and where the impressions are designed in notch form, and where the flanks of the notches are circularly convex with a radius of from 0.1 to 0.9 mm, preferably 0.5 mm, and/or the included angle of the notch is 45 to 135°, preferably 90°.

6. Process for the production of bioadhesive tablets comprising the mixing of at least one bioadhesive excipient and at least one lubricant and compression of a tablet, where the ratio- of lubricant to bioadhesive excipient is from 1:1300 to 1:1, and at least one surface of the tablet has concentric or parallel, straight-line and/or curved impressions.

7. Process for the production of bioadhesive tablets according to Claim 6, characterized in that the bioadhesive excipient is a substance which, on contact with the mucosa, develops an adhesive contact, such as a cellulose, a cellulose derivative, a carboxyvinyl polymer, a derivative of a carboxyvinyl polymer, a lectin or a natural substance or mixtures of these substances.

8. Process for the production of bioadhesive tablets according to Claim 6 or 7, characterized in that the lubricant makes the tableting of cohesive mixtures possible, such as talc, a metallic soap, a fatty acid or mixtures of these substances.

9. Process for the production of bioadhesive tablets according to at least one of Claims 6 to 8, characterized in that the content of bioadhesive excipient in the tablet is between 5 and 65% by weight, preferably between 10 and 45% by weight, and the content of lubricant is between 0.05 and 5% by weight, preferably between 1 and 3% by weight, and in that the ratio of lubricant to bioadhesive excipient is preferably from 1:10 to 1:15.

10. Process for the production of bioadhesive tablets according to at least one of Claims 6 to 9, characterized in that the surface has 2 to 12, preferably 3 to 5, concentric or parallel straight-line and/or curved impressions which have a depth of from 0.1 mm to 1.0 mm, preferably from 0.3 mm to 0.6 mm, calculated from the tablet surface, and have a distance apart of from 0.5 to 5 mm, preferably 1.5 to 2.5 mm, and where the impressions are designed in notch form, and where the flanks of the notches are circularly convex with a radius of from 0.1 to 0.9 mm, preferably 0.5 mm, and/or the included angle of the notch is 45 to 135°, preferably 90°.

11. Medicinal product in the form of a bioadhesive tablet according to Claim 1, containing as active ingredient antirheumatics, analgesics, antiparkinson agents, β-receptor blockers, sex hormones, contraceptives, cardiovascular agents, sleep hormones and pituitary hormones, antidiabetics, immuno-therapeutics or anticoagulants.

## Revendications

1. Comprimé bioadhésif, contenant au moins un matériau auxiliaire bioadhésif et au moins un agent lubrifiant, dans lequel le rapport de l'agent lubrifiant au matériau auxiliaire bioadhésif est de 1:1300 jusqu'à 1:1 et au moins une surface du comprimé présente des creux rectilignes et/ou incurvés concentriques ou s'étendant en parallèle.

2. Comprimé bioadhésif selon la revendication 1, caractérisé en ce que le matériau auxiliaire bioadhésif est une substance qui, par contact avec la muqueuse, développe un contact adhésif, notamment une cellulose, un dérivé de cellulose, un polymère carboxyvinylique, un dérivé d'un polymère carboxyvinylique, une lectine ou une substance naturelle ou des mélanges de ces substances.

3. Comprimé bioadhésif selon la revendication 1 ou 2, caractérisé en ce que l'agent lubrifiant permet le pastillage de mélanges cohésifs, notamment le talc, un savon métallique, un acide gras ou des mélanges de ces substances.

4. Comprimé bioadhésif selon au moins l'une des revendications précédentes, caractérisé en ce que la teneur en matériau auxiliaire bioadhésif du comprimé se situe entre 5% et 65% en poids, de préférence entre 10% et 45% en poids, et la teneur en agent lubrifiant se situe entre 0,05% et 5% en poids, de préférence entre 1% et 3% en poids, et le rapport de l'agent lubrifiant au matériau auxiliaire bioadhésif est de préférence de 1:10 jusqu'à 1:15.

5. Comprimé bioadhésif selon au moins l'une des revendications précédentes, caractérisé en ce que la surface présente 2 à 12, de préférence 3 à 5, creux rectilignes et/ou incurvés concentriques ou s'étendant en parallèle, qui ont une profondeur de 0,1 mm à 1,0 mm, de préférence de 0,3 mm à 0,6 mm, calculée à partir de la surface du comprimé, et une distance mutuelle de 0,5 à 5 mm, de préférence de 1,5 à 2,5 mm, et dans lequel les creux se présentent sous la forme d'encoches et les flancs des encoches sont incurvés en cercle d'un rayon de 0,1 à 0,9 mm, de préférence de 0,5 mm, et/ou l'angle d'ouverture de l'encoche est de 45 à 135°, de préférence de 90°.

6. Procédé de fabrication de comprimés bioadhésifs, comprenant le mélange d'au moins un matériau auxiliaire bioadhésif et d'au moins un agent lubrifiant et la compression d'un comprimé, dans lequel le rapport de l'agent lubrifiant au matériau auxiliaire bioadhésif est de 1:1300 jusqu'à 1:1 et au moins une surface du comprimé présente des creux rectilignes et/ou incurvés concentriques ou s'étendant en parallèle.

7. Procédé de fabrication de comprimés bioadhésifs selon la revendication 6, caractérisé en ce que le matériau auxiliaire bioadhésif est une substance qui, par contact avec la muqueuse, développe un contact adhésif, notamment une cellulose, un dérivé de cellulose, un polymère carboxyvinylique, un dérivé d'un polymère carboxyvinylique, une lectine ou un matériau naturel ou des mélanges de ces substances.

8. Procédé de fabrication de comprimés bioadhésifs selon la revendication 6 ou 7, caractérisé en ce que l'agent lubrifiant permet le pastillage de mélanges cohésifs, notamment le talc, un savon métallique, un acide gras ou des mélanges de ces substances.

9. Procédé de fabrication de comprimés bioadhésifs selon au moins l'une des revendications 6 à 8, caractérisé en ce que la teneur en matériau auxiliaire bioadhésif du comprimé se situe entre 5% et 65% en poids, de préférence entre 10% et 45% en poids, et la teneur en agent lubrifiant se situe entre 0,05% et 5% en poids, de préférence entre 1% et 3% en poids, et le rapport de l'agent lubrifiant au matériau auxiliaire bioadhésif est de préférence de 1:10 jusqu'à 1:15.

10. Procédé de fabrication de comprimés bioadhésifs selon au moins l'une des revendications 6 à 9, caractérisé en ce que la surface présente 2 à 12, de préférence 3 à 5, creux rectilignes et/ou incurvés concentriques ou s'étendant en parallèle, qui ont une profondeur de 0,1 mm à 1,0 mm, de préférence de 0,3 mm à 0,6 mm, calculée à partir de la surface du comprimé, et une distance mutuelle de 0,5 à 5 mm, de préférence de 1,5 à 2,5 mm, et dans lequel les creux se présentent sous la forme d'encoches et les flancs des encoches sont incurvés en cercle d'un rayon de 0,1 à 0,9 mm, de préférence de 0,5 mm, et/ou l'angle d'ouverture de l'encoche est de 45 à 135°, de préférence de 90°.

11. Médicament sous la forme d'un comprimé bioadhésif selon la revendication 1, contenant comme principe actif des anti-rhumatismaux, des analgésiques, des agents anti-maladie de Parkinson, des agents bloquants de β-récepteurs, des hormones sexuelles, des contraceptifs, des agents de circulation cardiaques, des hormones du sommeil et des hormones hypophysaires, des antidiabétiques, des agents immunothérapeutiques ou des anticoagulants.
